# EUROPEAN PATENT SPECIFICATION

(11) **EP 2 337 567 B1**
(45) Date of publication and mention of the grant of the patent: **31.12.2014**
(21) Application number: 09788414.2
(22) Date of filing: 05.08.2009
(51) Int. Cl.: A61K 31/522, A61P 17/12, A61K 47/10, A61K 47/26, A61K 47/38, A61K 47/20, A61K 47/32, A61K 9/08, A61K 47/06

(54) **VETERINARY PREPARATION**
VETERINÄRMEDIZINISCHE ZUBEREITUNG
PRÉPARATION VÉTÉRINAIRE

(30) Priority: 12.08.2008 PL 38586808
(43) Date of publication of application: 29.06.2011
(73) Proprietor: Przedsiebiorstwo Wielobranzowe "Vet-Agro" Sp. Z O.O., 20-616 Lublin (PL)
(72) Inventor: SIWICKI Andrzej Krzysztof, PL-05-500 Piaseczno (PL); GLUSZAK Jan, PL-21 -030 Motycz 102c (PL)
(74) Representative: Hladyniuk, Jozef Wawrzyniec
(86) International application number: PCT/PL2009/000079
(87) International publication number: WO 2010/019059

(56) References cited:
- WO-A1-03/013544
- IT-A1- MI 932 019
- KUIE TAY S: "EFFICACY OF INOSINE PRANOBEX ORAL THERAPY IN SUBCLINICAL HUMAN PAPILLOMAVIRUS INFECTION OF THE VULVA: A RANDOMIZED DOUBLE-BLINDED PLACEBO CONTROLLED STUDY" INTERNATIONAL JOURNAL OF STD AND AIDS, vol. 7, no. 4, 1 July 1996 (1996-07-01), pages 276-280, XP000676476 ROYAL SOCIETY OF MEDICINE SERVICES, LONDON, GB ISSN: 0956-4624
- ANONYMOUS: "VIRUXAN" INTERNET ARTICLE, [Online] 18 February 2005 (2005-02-18), pages 1-7, XP002552362 Retrieved from the Internet: URL:http://web.archive.org/web/20050218061 908/http://www.carloanibaldi.com/terapia/s chede/VIRUXAN.htm#SottoTitolo_3> [retrieved on 2009-10-26]
- PENNA, C. ET AL.: "Utilizzazione del metisoprinolo (Viruxan) per via topica nella condilomatosi genitale femminile" MINERVA GINECOLOGICA, vol. 41, no. 1, 1989, pages 27-32, XP009124812
- ELO, J. & MATE, Z.: "Combined therapy with isoprinosine and CO2 laser microsurgery for the treatment of laryngeal papillomatosis" ARCH OTORHINOLARYNGOL, vol. 244, 1988, pages 342-345, XP009124777
- PATEL P. ET AL.: "Inosine pranobex in recurrent laryngeal papillomatosis" THE JOURNAL OF LARYNGOLOGY AND OTOLOGY, vol. 101, 1987, pages 1306-1307, XP000912480
- BENTON, E.C. ET AL.: "Trial of inosine pranobex in the management of cutaneous viral warts" JOURNAL OF DERMATOLOGICAL TREATMENT, no. 1, 1991, pages 295-297, XP009124779

## Description

The object of the invention is a veterinary preparation intended as an aid in therapeutic procedure.

Pharmacology offers a preparation named methisoprinol^{USL}, which is a synthetic compound of inosine nucleoside and 1-dimethylamino-2-propanol (DIP) 4-(acetylamino) benzoate(PacBA). It shows a strong immunomodulating and antiviral effects. It activates non-specific humoral and cellular mechanisms. It increases cytotoxic lymphocyte activity, stimulates proliferation of lymphocytes and microphage activity. It increases the level of interleukin 1 (IL 1), interleukin 2 (OL2) and interleukin 12 (IL 12). It inhibits autoimmunoagression processes, decreases the effect of immunosuppressants and counteracts excessive humoral immunological reactions. The preparation is applied only to humans. Due to a large daily dose of about 4g (4 x 1g), coupled with a several-month long duration of the treatment, the preparation is manufactured only in the form of tablets. The dose size is dictated by the fact that after administration the preparation is active for only a few minutes.

The patent dossier PL 194749 B1, in turn, presents a ferric preparation which contains 10-20 parts by weight of iron dextrane and, as an additional ingredient, 1-20 parts by weight of methisoprinol. This preparation is intended for prophylaxis or treatment of anaemia in piglets, in form *pro injectio.*

The publication "Isoprinosine: pharmacological and toxicological properties in animals" (T. Ginssberg, L.N. Simon and A.J. Glasky, 7th International Congress of Pharmacology, Paris, July 16/2/1978) provides a description of a research on the toxicity of methisoprinol in animals. Apart from the aforementioned ferric preparation, so far, methisoprinol has not been utilised in any other veterinary medicinal preparation.

It is known that water solutions of isoprinosine are bitter, which makes voluntary oral administration of the medicinal preparation difficult for both humans and animals.

The patent application IT MI 932019 A1 reports experiments where eye ointment contains 1,5% by weight of methisoprinol is administered to rabbits in treatment of herperic keratitis.

The crucial characteristic of the veterinary preparation, which contains methisoprinol as an active substance, excipients and, optionally, additional ingredients, is that the preparation contains from 15 to 30% by weight of methisoprinol.
Methisoprinol is preferably dissolved in water. In another preferable variant, methisoprinol is suspended in polyhydric alcohol, where the alcohol is preferably propylene glycol or sorbitol. The excipient is preferably a preservative or/and antiseptic, especially phenol.
The additional ingredient is preferably a gelling agent or/and thickening agent. Particularly effective additional ingredients here are cellulose derivatives.

The contemporary medical research focuses mainly on either synergism of the already existing substances or development of their better "isomers". Both processes are long-lasting, costly and they do not guarantee success.

Unexpectedly, it has been proven that it is possible to utilize methisoprinol in veterinary medicine as an active substance on a large scale. Moreover, the suggested forms of the medicinal preparation do not cause stress in animals during its administration, as is happens the case in injection. The new preparations are easily administered orally to animals, both in its basic form as well as added to feed.

The preparation and its applications are presented in the following examples:

### Example I. Restorative suspension for animals

| | | | | | |
|---|---|---|---|---|---|
| Composition: | isoprinosine | | . | . | 25.0% w/v |
| | phenol | . | . | . | 0.5% w/v |
| | polivinylopyrrolidon | | | . | 3% |
| | propylene glycol | | . | . | ad 100% w/v |

Recommendation: as an immunomodulating preparation, especially as the first dose in a treatment of severely weakened animals or for animals to be transported. Route of administration: oral.

### Example II. Syrup

| | | | | | |
|---|---|---|---|---|---|
| Composition: | isoprinosine | | . | . | 30% w/v |
| | sorbitol 70% | . | . | . | ad 100% |

Recommendation and route of administration: see Example 1.

### Example III. Syrup

| | | | | | |
|---|---|---|---|---|---|
| Composition: | isoprinosine | | . | . | 20% w/v |
| | guar gum | . | . | . | 10% |
| | water | . | . | . | ad 100% |

Recommendation and route of administration: see Example I.

### Example IV. Syrup

| | | | | | |
|---|---|---|---|---|---|
| Composition: | isoprinosine | | . | . | 20% w/v |
| | hydroxyethyl cellulose | | | . | 1% |
| | water | . | . | . | ad 100% |

Recommendation and route of administration: see Example 1.

In order to obtain a preparation in accordance with the patent dossier, the desired portion of methisoprinol is dissolved in water or its solution is prepared in the standard way. Next, the additional ingredients are added and the solution is diluted to volume and undergoes the process of sterile filtration. The preparation can be utilized directly after being manufactured or batched and packed in a standard way.

### Example V.

Fish feed was saturated with the preparation by spraying, in accordance with Example 1, dried and fed to a test group of rainbow trout. At the same time, a control group of rainbow trout was fed with the same amount of feed but not enriched with the preparation. The enriched feed, containing 80mg/kg m.c, was administered for three days. Both the test and control groups were experimentally infected with pathogenic viruses. After the three days of treatment, the number of dead fish in the test group was 20% lower.

### Example VI.

Before transportation, 12 recreational horses were divided into two groups of 6. the horses from the one (test) group were orally administered with the veterinary medicinal preparation in the dose of 100mg/kg m.c., in accordance with Example 2. The horses were transported to the distance of about 110km on lorries/trailers designed for that purpose. After 7 days from the day of transportation, 1 horse from the control group was diagnosed with conjunctivitis and catarrh; two other horses from the same group were diagnosed only with conjunctivitis and one horse only with catarrh. In turn, none of the horses from the test group showed any symptoms of the two conditions.

## Claims

1. Oral veterinary preparation containing methisoprinol as an active substance, excipient and, optionally, additional ingredients wherein the composition contains from 15 to 30% by weight of methisoprinol which is dissolved in water or is suspended in polyhydric alcohol.

2. Preparation according to claim 1 wherein the polyhydric alcohol is propylene glycol or sorbitol.

3. Preparation according to claim 1 wherein the excipient is a preservative or/and antiseptic.

4. Preparation according to claim 3 wherein the preservative and/or antiseptic is phenol.

5. Preparation according to claim 1 wherein the additional ingredient is a gelling agent or/and thickening agent.

6. Preparation according to claim 5 wherein the additional ingredient is a cellulose derivative.

7. Use of a veterinary preparation according to claims 1 - 6 in the manufacture of a medicament for the treatment of severely weakened animals.

## Patentansprüche

1. Perorales das Methisoprinol als einen Wirkstoff, Zusatzstoffe und eventuell akzessorische Substanzen enthaltende Veterinärpräparat, **dadurch gekennzeichnet, dass** die Zusammensetzung gewichtsmäßig von 15 bis 30% des im Wasser gelösten oder im Polyol suspendierten Methisoprinols in sich einschließt.

2. Präparat nach Anspruch 1 **dadurch gekennzeichnet, dass** als Polyol Propylenglykol oder Sorbitol verwendet werden.

3. Präparat nach Anspruch 1 **dadurch gekennzeichnet, dass** es ein Konservierungsmittel oder/und Antiseptikum als eine Zusatzsubstanz enthält.

4. Präparat nach Anspruch 3 **dadurch gekennzeichnet, dass** als Konservierungsmittel oder/und Antiseptikum Phenol auftritt.

5. Präparat nach Anspruch 1 **dadurch gekennzeichnet, dass** es in sich eine Gelierungs- oder/und Verdickungssubstanz als eine akzessorische Substanz einschließt.

6. Präparat nach Anspruch 5 **dadurch gekennzeichnet, dass** die Gelierungs- oder/und Verdickungssubstanz ein Cellulosederivat darstellt.

7. Verwendung des Veterinärpräparates nach Ansprüchen 1 - 6 zur Herstellung des Heilmittels für stark abgeschwächte Tiere.

## Revendications

1. Composition vétérinaire orale contenant méthisoprinol en tant que principe actif, des excipients et, éventuellement, des additifs, ladite composition **caractérisée en ce qu'**elle contient de 15% à 30 % en poids de méthisoprinol qui est dissous dans l'eau ou suspendu dans un polyalcool.

2. Compositon selon la revendication 1 **caractérisée en ce que** ledit polyalcool est le propylène glicol ou le sorbitol.

3. Compositon selon la revendication 1 **caractérisée en ce qu'**elle contient en tant qu'excipient un conservateur ou un antiseptique.

4. Compositon selon la revendication 3 **caractérisée en ce que** ledit conservateur ou/et antiseptique est le phénol.

5. Compositon selon la revendication 1 **caractérisée en ce qu'**elle contient en tant qu'additif un agent gélifiant ou/et un agent épaississant.

6. Compositon selon la revendication 5 **caractérisée en ce que** ledit agent gélifiant ou/et agent épaississant est un dérivé de cellulose.

7. Utilisation de la composition vétérinaire selon les revendications 1-6 pour fabriquer une préparation destinée à soigner des animaux très affaiblis.
